# EUROPEAN PATENT SPECIFICATION

(11) **EP 3 842 110 B1**
(45) Date of publication and mention of the grant of the patent: **08.02.2023**
(21) Application number: 19306757.6
(22) Date of filing: 23.12.2019
(51) Int. Cl.: B01D 19/04, C07C 29/34, C07C 31/125

(54) **FOAM DESTABILIZERS**
SCHAUMDESTABILISATOREN
DÉSTABILISATEURS EN MOUSSE

(43) Date of publication of application: 30.06.2021
(73) Proprietor: Oleon N.V., 9940 Ertvelde (BE)
(72) Inventor: KERBRAT, Marion, 60280 Margny-les-Compiegne (FR); PACKET, Dirk, 3110 Rotselaar (BE); DE SCHRIJVER, Bert, 9270 Kalken (BE); ZITOUNI, Karima, 91420 MORANGIS (FR)
(74) Representative: Santarelli

(56) References cited:
- EP-A1- 2 913 319
- US-A- 4 421 666

## Description

The present invention relates to specific alcohols and their use as antifoam and/or defoamer.

A foam is a dispersion of gas bubbles into a liquid.

Almost every industrial sectors are concerned by unwanted foam formation, such as food processing, paper industry, textile drying, paint manufacture, oil and gas industry and waste water treatment.

Unwanted foam can be formed during an industrial process, such as during the agitation of a composition comprising a compound capable of foaming the composition. This production of unwanted foam may lead to significant problems, such as reductions in product performance or productivity losses.

Thus, it is necessary to prevent or reduce the foaming, or to break the foam.

Antifoams and defoamers are chemical compounds used for foam control. They both destabilize a foam.

An antifoam prevents or reduces the foam formation.

An defoamer breaks an existing foam.

Various antifoams and defoamers are currently available.

US 2014/0251605 A1 describes a method for defoaming a foam with a composition comprising at least an alcohol, linear or branched, represented by Figures I to IV. Based on those figures, the alcohol can potentially comprise between 3 and 997 carbon atoms. Further relevant prior art is disclosed in EP2913319 and US4421666.

It has been surprisingly found that specific branched primary alcohols present better destabilization of foam properties than any other alcohols, allowing reducing foam formation and/or breaking a foam faster.

The present invention therefore relates to an use of a branched primary alcohol of Formula (I), 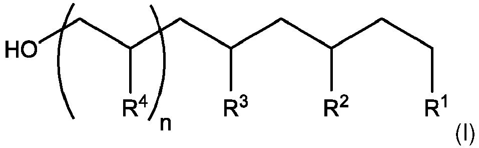 wherein :
- n = 0 or 1;
- R¹,R², R³ and R⁴ are linear or branched alkyl groups, identical or different, comprising from 1 to 6 carbon atoms;
as an antifoam and/or a defoamer.

A alkyl group is a group consisting of carbon and hydrogen atoms.

Preferably, the alkyl groups R¹,R², R³ and R⁴ are saturated.

The alkyl groups may be a methyl, ethyl, propyl, butyl, pentyl or hexyl group, or any of their isomers.

Preferably, the branched primary alcohol of Formula (I) is saturated.

Advantageously, in use according to the invention, when n=0, the branched primary alcohol is a primary alcohol trimer, and when n=1, the branched primary alcohol is a primary alcohol tetramer, both being obtainable by the condensation of respectively three and four primary alcohol monomers according to a Guerbet reaction.

The Guerbet reaction is well known in the art. The Guerbet reaction comprises the condensation of two, identical or different, primary alcohol monomers with elimination of water. It typically proceeds in the presence of a base such as NaOH or KOH and/or a catalyst such as palladium, at a temperature of at least 120°C, to form a primary alcohol dimer which is a beta-alkylated primary alcohol.

However, the resulting primary alcohol dimer, usually called Guerbet alcohol, can further react with a primary alcohol monomer to form higher primary alcohol oligomers, such as a primary alcohol trimer and/or a primary alcohol tetramer, usually a mixture of at least a primary alcohol trimer and a primary alcohol tetramer is obtained.

The primary alcohol monomer may be linear or branched.

Preferably the primary alcohol monomer is not substituted by any heteroatom other than the oxygen of the hydroxyl group.

Preferably, the primary alcohol monomer is saturated.

Preferably, branched primary alcohol(s) of Formula (I) is/are obtainable from primary alcohol monomer(s) chosen from the group consisting of butanol, 2-methylpropanol, pentanol, 3-methylbutanol, 2-methylbutanol, 3-methylpentanol, 4-methylpentanol, octanol and their mixtures.

In case of at least two different primary alcohol monomers are used, then the resulting primary alcohol oligomers may be a mixture of primary alcohol trimers and/or a mixture of primary alcohol tetramers, with a least one alkyl group R¹,R², R³, R⁴, different from the others.

Advantageously, in use according to the invention, branched primary alcohols of Formula (I) with n=0 and with n=1, are used.

As an example, this corresponds to a mixture of primary alcohol trimer(s) and primary alcohol tetramer(s) such as obtained by condensation according to a Guerbet reaction as mentioned above.

Preferably, the ratio (total quantity of branched primary alcohol(s) of Formula (I) with n=0) / (total quantity of branched primary alcohol(s) of Formula (I) with n=1) is comprised between 0.1 and 10, more preferably between 1 and 6, even more preferably between 1.5 and 4.

By "total quantity of branched primary alcohol(s) of Formula (I) with n=0", it is intended the weight of all branched primary alcohol(s) of Formula (I) with n=0 molecules.

By "total quantity of branched primary alcohol(s) of Formula (I) with n=1", it is intended the weight of all branched primary alcohol(s) of Formula (I) with n=1 molecules.

Preferably, in use according to the invention, R¹, R², R³ and Rare identical.

In case of the primary alcohol monomers used to prepare the branched primary alcohol(s) of Formula (I) via a Guerbet reaction are identical, then a primary alcohol trimer and/or a primary alcohol tetramer, with all alkyl group R1 ,R2, R3, R4, identical is/are formed.

Alternatively, in use according to the invention, R1, R2, R3 and R4 are independently chosen among two alkyl groups.

As an example, this corresponds to a mixture of primary alcohol trimers and/or primary alcohol tetramers such as obtained by condensation according to a Guerbet reaction starting from two different primary alcohol monomers as mentioned above.

Advantageously, in use of the invention, R1, R2, R3 and R4, individually, comprise between 2 and 4 carbon atoms.

Preferably, branched primary alcohol(s) of Formula (I) is/are obtainable from primary alcohol monomer(s) chosen from the group consisting of butanol, pentanol, 3-methylbutanol and their mixtures.

Advantageously, a branched primary alcohol of Formula (I), destabilize a foam, in particular a foam in formation or already formed on an aqueous composition.

An effective amount of branched primary alcohol of Formula (I), sufficient to destabilize a foam, is preferably comprised between 100 ppm and 5000 ppm, more preferably between 150 ppm and 3000 ppm, even more preferably between 200 and 2000 ppm.

The person skilled in the art knows how to adapt the amount regarding the quantity and the type of compound capable of foaming the composition.

A branched primary alcohol of Formula (I) reduces the foam formation. More particularly, the foam formation is immediately reduced by at least 50% and the foam formed collapsed within 5 min, as showed in Example 2.3.

The disclosure also relates to a composition comprising a branched primary alcohol of Formula (I) and water.

The branched primary alcohol of Formula (I) is as described above, including preferential and advantageous features.

The total quantity of branched primary alcohol(s) of Formula (I) is of at least 0.01% by weight, more preferably at least 0.015% by weight, even more preferably at least 0.02% by weight based on the weight of the composition.

The composition may further comprise a compound capable of foaming the composition.

The compound capable of foaming the composition may be any residue of an industrial process that induce foam formation, such as a surfactant used in lubrication or in emulsification.

The quantity of compound capable of foaming the composition is preferably of at least 0.05 % by weight, more preferably of at least 0.1 % by weight, based on the weight of the composition.

Preferably, the quantity of compound capable of foaming the composition is at most 5% by weight, more preferably at most 1% by weight, even more preferably at most 0.5% by weight based on the weight of the composition.

Water may be tap water, distilled water or a brine.

The quantity of water is preferably of at least 50% by weight based on the weight of the composition.

The composition of invention may further comprise a beta-alkylated primary alcohol comprising from 6 to 16 carbon atoms.

The beta-alkylated primary alcohol is preferably a primary alcohol dimer obtained by a Guerbet reaction.

Preferably, the ratio (total quantity of beta-alkylated primary alcohol(s) comprising from 6 to 16 carbon atoms) / (total quantity of branched primary alcohol of Formula (I)) is comprised between 0 and 9, more preferably between 0 and 6.

By "total quantity of beta-alkylated primary alcohol(s) comprising from 6 to 16 carbon atoms" it is intended the weight of all beta-alkylated primary alcohol molecules comprising from 6 to 16 carbon atoms.

Preferably, the beta-alkylated primary alcohol comprises from 8 to 12 carbon atoms.

Preferably, the ratio (total quantity of beta-alkylated primary alcohol(s) comprising from 8 to 12 carbon atoms) / (total quantity of branched primary alcohol of Formula (I)) is comprised between 0 and 9, more preferably between 0 and 6.

By "total quantity of beta-alkylated primary alcohol(s) comprising from 8 to 12 carbon atoms" it is intended the weight of all beta-alkylated primary alcohol molecules comprising from 8 to 12 carbon atoms.

A process for preparing the composition of the invention comprises a step of bringing together a branched primary alcohol of Formula (I), water, and optionally a compound capable of foaming the composition and a beta-alkylated primary alcohol comprising from 6 to 16 carbon atoms.

A mixture comprises or consists of :
- at least 25% by weight of :
   - branched primary alcohol(s) of Formula (I) with n=0; and
   - branched primary alcohol(s) of Formula (I) with n=1;
- less than 75% by weight of beta-alkylated primary alcohol(s) comprising from 6 to 16 carbon atoms.
A preferred mixture comprises or consists of :
- at least 50% by weight of
   - branched primary alcohol(s) of Formula (I) in which n=0; and
   - branched primary alcohol(s) of Formula (I) in which n=1;
- less than 50% by weight of beta-alkylated primary alcohol(s) comprising from 8 to 12 carbon atoms.

Preferably, in mixtures of the invention, the branched primary alcohol(s) of Formula (I) and the beta-alkylated primary alcohol(s) are obtainable by a Guerbet reaction.

In mixtures of the invention, the ratio (total quantity of beta-alkylated primary alcohol(s) comprising from 6 to 16 carbon atoms) / (total quantity of branched primary alcohol of Formula (I)) is preferably comprised between 0 and 9, more preferably between 0 and 6.

More preferably the ratio (total quantity of beta-alkylated primary alcohol(s) comprising from 8 to 12 carbon atoms) / (total quantity of branched primary alcohol of Formula (I)) is comprised between 0 and 9, more preferably between 0 and 6.

In mixtures of the invention, the ratio (total quantity of branched primary alcohol(s) of Formula (I) with n=0) / ratio (total quantity of branched primary alcohol(s) of Formula (I) with n=1) is preferably comprised between 0.1 and 10, more preferably between 1 and 6, even more preferably between 1.5 and 4.

The invention also relates to a method for destabilizing a foam of a composition by contacting the foam with at least a branched primary alcohol of Formula (I) or a mixture of the invention.

The contact between the foam and the branched primary alcohol(s) of Formula (I) may be achieved by adding the branched primary alcohol(s) of Formula (I) on the surface of the foam or by adding it into the composition generating the foam.

The invention further relates to a method for reducing foam formation of a composition, by adding into the composition at least a branched primary alcohol of Formula (I) or a mixture of the invention.

The invention also relates to a method for breaking a foam of a composition, by contacting the foam with at least a branched primary alcohol of Formula (I) or a mixture of the invention.

The contact between the foam and the branched primary alcohol(s) of Formula (I) may be achieved by adding the a branched primary alcohol(s) of Formula (I) on the surface of the foam or by adding it into the composition generating the foam.

In methods according to the invention, the branched primary alcohol(s) of Formula (I) and the mixture of the invention, are as described above, including preferential and advantageous features.

Preferably, a mixture of branched primary alcohols of Formula (I) with n=0 and with n=1, are used in methods according to the invention.

In particular, a mixture of primary alcohol trimer(s) and primary alcohol tetramer(s) such as obtained by condensation according to a Guerbet reaction are used.

Advantageously, in methods of the invention, the total quantity of branched primary alcohol(s) of Formula (I) is of at least 0.01% by weight based on the weight of the composition.

Preferably, the total quantity of branched primary alcohol(s) of Formula (I) represents at least 0.015% by weight, even more preferably at least 0.02% by weight based on the weight of the composition.

Preferably, the total quantity of branched primary alcohol(s) of Formula (I) represents at most 0.5% by weight based on the weight of the composition.

Preferably, in methods according to the invention, the composition is a composition comprising water, in particular at a content of at least 50% by weight based on the weight of the composition.

The invention is further described in the following examples. It will be appreciated that the invention as claimed is not intended to be limited in any way by these examples.

### Example 1: Preparation of branched primary alcohol of Formula (I) and mixture according to the invention

### 1.1 Preparation of primary alcohol oligomers via a Guerbet reaction

A Guerbet reaction was conducted as described in patent US 4,518,810 from 3-methylbutanol as starting primary alcohol monomer, and using KOH as a base and palladium as catalyst. The reaction medium was heated up its boiling point.

The crude reaction mixture was washed several times with demineralized water to remove all of the soaps. The washed product was subsequently filtered and dried under vacuum. The remaining starting primary alcohol monomer was separated by distillation.

The primary alcohol dimer comprising 10 carbon atoms (2-isopropyl-5-methylhexanol) was then separated by distillation.

The distillate obtained comprised 99.7 wt% of primary alcohol dimer C10 and 0.3 wt% primary alcohol trimer comprising 15 carbon atoms, and corresponds to comparative mixture 1.

The residue ("first residue") was composed of 28.1 wt% of primary alcohol trimer C15, 11.5 wt% of primary alcohol tetramer C20, and 60.4 wt% of remaining primary alcohol dimer C10.

The remaining primary alcohol dimer was further distilled from this first residue.

The second residue obtained was then composed of 68.2 wt% of primary alcohol trimer C15, 31.2 wt% of primary alcohol tetramer C20, and 0.6 wt% of primary alcohol dimer C10.

### 1.2 Preparation of mixtures 1-5

The second residue obtained in Example 1.1 corresponds to mixture 1 according to the invention.

Mixture 2 according to the invention was obtained by diluting 75 wt% of mixture 1 with 25 wt% of comparative mixture 1.

The first residue obtained in Example 1.1 corresponds to mixture 3.

Mixtures 4-5 were obtained by diluting mixture 3 with respectively 25 wt% and 50 wt% of comparative mixture 1.

Contents of each mixture 1-4 according to the invention and of comparative mixture 1 are described in Table 1 below.

**Table 1: Contents of mixtures 1-4 according to the invention and of mixture 5 according to the use of the invention and of comparative mixture 1**

| | Primary alcohol dimer C10 (wt%*) | Primary alcohol trimer C15 (wt%*) | Primary alcohol tetramer C20 (wt%*) | Total quantity of alcohols of Formula (I) (wt%) |
|---|---|---|---|---|
| Mixture 1 | 0.6 | 68.2 | 31.2 | 99.4 |
| Mixture 2 | 25.4 | 51.2 | 23.4 | 74.6 |
| Mixture 3 | 60.4 | 28.1 | 11.5 | 39.6 |
| Mixture 4 | 70.3 | 21.1 | 8.6 | 29.7 |
| Mixture 5 | 80.0 | 14.2 | 5.8 | 20.0 |
| Comparative mixture 1 | 99.7 | 0.3 | 0 | 0.3 |

| | | | | |
|---|---|---|---|---|
| *based on the weight of the mixture | | | | |

### Example 2: Foaming characteristics of compositions according to the invention

### 2.1 Preparation of compositions 1-5

Compositions 1-5 were prepared by adding 0.5 wt% of a foaming agent (sodium dodecylbenzene sulfonate) and respectively 0.1 wt% of mixture 1-5, into tap water; weight percentages being based on the weight of each composition.

### 2.2 Preparation of comparative composition 1

Comparative composition 1 was prepared as compositions of the invention, by adding 0.5 wt% of a foaming agent (sodium dodecylbenzene sulfonate), and 0.1 wt% of comparative mixture 1 into tap water.

### 2.3 Foam stability

The method used to determine the efficiency of the specific alcohols to destabilize foam is based on ASTM D892.

A blank sample was prepared by adding 0.5 wt% of a foaming agent (sodium dodecylbenzene sulfonate), into tap water, weight percentage being based on the weight of the blank sample.

200 mL of each sample (compositions of the invention, comparative composition and blank), were respectively introduced in a 1000 mL graduated cylinder. Each cylinder was then placed in a bath at 24°C. Air was injected in each sample at a flow rate of 94 mL/min for 5 minutes and then the samples were allowed to settle for 10 minutes.

The volumes of foam were measured just after the air flow stopped (t=0), and monitored for the 10 minutes period.

Results are summarized in Table 2 below:

**Table 2: Evolution of volumes of foam of compositions 1-4 of the invention and of composition 5 according to the use of the invention and the comparative composition**

| | | Volumes of foam (mL) | | | | |
|---|---|---|---|---|---|---|
| | Antifoam | 0 min | 2 min | 4 min | 5 min | 10 min |
| Blank | - | 580 | 550 | 535 | 530 | 480 |
| Composition 1 | 0.1% of mixture 1 | 115 | 0 | 0 | 0 | 0 |
| Composition 2 | 0.1% of mixture 2 | 115 | 0 | 0 | 0 | 0 |
| Composition 3 | 0.1% of mixture 3 | 240 | 50 | 0 | 0 | 0 |
| Composition 4 | 0.1% of mixture 4 | 265 | 70 | 0 | 0 | 0 |
| Composition 5 | 0.1% of mixture 5 | 285 | 165 | 10 | 0 | 0 |
| Comparative composition 1 | 0.1% of comparative mixture 1 | 350 | 215 | 60 | 35 | 20 |

It can be observed with the blank sample, that without any antifoam, a lot of foam is produced: 580 mL of foam at t=0 and still 480 mL after 10 min of rest.

When a mixture of the invention is added to the aqueous composition comprising a foaming agent, then foam formation is reduced by at least 50% (volume of foam at t=0 of composition 5 is of 285 mL) to up to 80% (volume of foam at t=0 of composition 2 is of 115 mL).

It can also be observed that globally the more the total quantity of alcohols of Formula (I), the lower the foaming and the faster the foam formed disappears.

More particularly, the foam formed is not stable and disappears in 4 min by using 0.1% of mixture 4, *e.g.* 0.03 wt% of alcohols of Formula (I); and faster, in 2 min, by using 0.1% of mixture 2, *e.g.* 0.07 wt% of alcohols of Formula (I).

All those results demonstrate that the alcohols of Formula (I) and the mixtures of the invention comprising them, can be used as a good antifoam.

### 2.4 Defoaming property of composition 1 according to the invention

The method used to determine the efficiency of the mixture of the invention as defoamer is based on ASTM D892.

Two samples of 200 mL of tap water comprising 0.5 wt% of a foaming agent (sodium dodecylbenzene sulfonate) were each placed into a 1000 mL graduated cylinder which were then placed in a bath at 24°C.

Air was injected into each sample at a flow rate of 94 mL/min for 5 minutes. Once the injection of air stopped, 0.1 wt% of mixture 1 according to the invention was added on the foam formed of one sample, to form composition 1 of the invention.

All weight percentage are based on the weight of the corresponding composition. The sample which do not received mixture of the invention corresponds to the blank.

The volume of foam of blank sample was measured just after the air flow stopped (t=0) and for 10 minutes.

The volume of foam of composition 1 was measured just after addition of mixture 1 (t=0), and monitored for 10 minutes.

Data are gathered in Table 3 below.

**Table 3: Defoaming properties of composition 1 of the invention**

| | | Volumes of foam (mL) | | | |
|---|---|---|---|---|---|
| | Defoamer | 0 min | 3 min | 5 min | 6 min |
| Blank | - | 600 | 550 | 540 | 530 |
| Composition 1 | 0.1% of mixture 1 | 600 | 120 | 0 | 0 |

It can be seen that composition 1 of the invention comprising 0.1 wt% of mixture 1 of the invention, can break an existing foam within 5 minutes.

Branched primary alcohols of Formula (I), in particular a mixture of a branched primary alcohol comprising 15 carbon atoms and of a branched primary alcohol comprising 20 carbon atoms, both obtained from condensation of 3-methylbutanol, may be used as a defoamer.

### Example 3: Foaming characteristics of comparative alcohols

### 3.1 Alcohols wich are not of Formula (I)

- 2-ethylhexanol from Perstorp;
- Hexyldecanol, Isofol 16 from Sasol;
- 2-isopropyl-5-methylhexanol prepared in Example1.1.

### 3.2 Preparation of comparative compositions 2-4

Comparative compositions 2-4 were prepared by adding 0.5 wt% of a foaming agent (sodium dodecylbenzene sulfonate) and respectively 0.1 wt% of 2-ethylhexanol, hexyldecanol, a mixture of 60% of 2-isopropyl-5-methylhexanol and 40% of hexyldecanol, into tap water; weight percentages being based on the weight of each composition.

### 3.3 Foaming characteristics of comparative compositions 2-4

The method used to measure volumes of foam of different comparative compositions 2-4 is the same as method described in Example 2.3.

Results are given in Table 4 below.

**Table 4: Volumes of foam of comparative compositions 2-4**

| | | Volume of foam (mL) | |
|---|---|---|---|
| | Antifoam | 0 min | 10 min |
| Blank | - | 580 | 490 |
| Comparative composition 2 | 0.1% of 2-ethylhexanol | 470 | 43 |
| Comparative composition 3 | 0.1% of hexyldecanol | 570 | 430 |
| Comparative composition 4 | 0.1% of a mixture of 60% of 2-isopropyl-5-methylhexanol and 40% of hexyldecanol | 490 | 180 |

At t=0, the foam formation is only reduced by at most 19% (comparative composition 2). After 10 minutes, foam remains in each comparative compositions 2-4.

By comparing comparative composition 3 with compositions according to the invention, we can conclude that using a branched alcohol comprising 16 carbon atoms but which is not of Formula (I), doesn't reduce foaming as much as the alcohols of Formula (I).

2-ethylhexanol that is specifically cited and exemplified in patent US 2014/0251605 A1, reduces foaming by only 19% at t=0 and after 10 minutes, foam is still present above the composition, when used at 0.1wt%.

## Claims

1. Use of a branched primary alcohol of Formula (I), 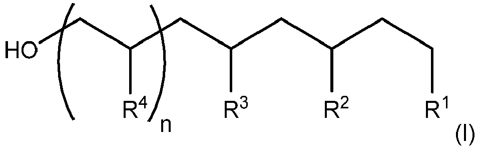 wherein :
- n = 0 or 1;
- R¹, R², R³ and R⁴ are linear or branched alkyl groups, identical or different, comprising from 1 to 6 carbon atoms;
as an antifoam and/or a defoamer.

2. Use according to claim 1, wherein when n=0, the branched primary alcohol is a primary alcohol trimer, and when n=1, the branched primary alcohol is a primary alcohol tetramer, both being obtainable by the condensation of respectively three and four primary alcohol monomers according to a Guerbet reaction.

3. Use according to claim 1 or 2, wherein branched primary alcohols of Formula (I) with n=0 and with n=1, are used.

4. Use according to any of claims 1 to 3, wherein R¹, R², R³ and R⁴ are identical.

5. Use according to any of claims 1 to 3, wherein R¹, R², R³ and R⁴ are independently chosen among two alkyl groups.

6. Use according to any of claims 1 to 5, wherein R¹, R², R³ and R⁴, individually, comprise between 2 and 4 carbon atoms.

7. Mixture comprising or consisting of:
- at least 25% by weight of :
- branched primary alcohol(s) of Formula (I) wherein n=0; and
- branched primary alcohol(s) of Formula (I) wherein n=1;
- less than 75% by weight of beta-alkylated primary alcohol(s) comprising from 6 to 16 carbon atoms.

8. Method for destabilizing a foam of a composition by contacting the foam with at least a branched primary alcohol of Formula (I) or a mixture according to claim 7.

9. Method for reducing foam formation of a composition, by adding into the composition at least a branched primary alcohol of Formula (I) or a mixture according to claim 7.

10. Method for breaking a foam of a composition, by contacting the foam with at least a branched primary alcohol of Formula (I) or a mixture according to claim 7.

11. Method according to any of claims 8 to 10, wherein the total quantity of branched primary alcohol(s) of Formula (I) is of at least 0.01% by weight based on the weight of the composition.

## Patentansprüche

1. Verwendung eines verzweigten primären Alkohols der Formel (I), 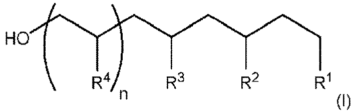 wobei:
- n = 0 oder 1;
- R¹, R², R³ und R⁴ identische oder unterschiedliche lineare oder verzweigte Alkylgruppen sind, die von 1 bis 6 Kohlenstoffatome umfassen;
als Antischaummittel und/oder Entschäumer.

2. Verwendung nach Anspruch 1, wobei, wenn n=0, der verzweigte primäre Alkohol ein primäres Alkohol-Trimer ist, und wenn n=1, der verzweigte primäre Alkohol ein primäres Alkohol-Tetramer ist, wobei beide durch die Kondensation von jeweils drei oder vier primären Alkohol-Monomeren entsprechend einer Guerbet-Reaktion erhältlich sind.

3. Verwendung nach Anspruch 1 oder 2, wobei die verzweigten primären Alkohole der Formel (I) mit n=0 und mit n=1 verwendet werden.

4. Verwendung nach einem der Ansprüche 1 bis 3, wobei R¹, R², R³ und R⁴ identisch sind.

5. Verwendung nach einem der Ansprüche 1 bis 3, wobei R¹, R², R³ und R⁴ unabhängig aus zwei Alkylgruppen ausgewählt werden.

6. Verwendung nach einem der Ansprüche 1 bis 5, wobei R¹, R², R³ und R⁴ einzeln zwischen 2 und 4 Kohlenstoffatome umfassen.

7. Gemisch, das Folgendes umfasst oder aus Folgendem besteht:
- mindestens 25 Gew.-% an:
- verzweigtem(-n) primärem (-n) Alkohol(en) der Formel (I), wobei n=0; und
- verzweigtem(-n) primärem (-n) Alkohol(en) der Formel (I), wobei n=1;
- weniger als 75 Gew.-% an beta-alkyliertem (-n) primärem (-n) Alkohol(en), 6 bis 16 Kohlenstoffatome umfassend.

8. Verfahren zum Destabilisieren von Schaum einer Zusammensetzung durch Kontaktieren des Schaums mit mindestens einem verzweigten primären Alkohol der Formel (I) oder einem Gemisch nach Anspruch 7.

9. Verfahren zum Verringern von Schaumbildung einer Zusammensetzung durch Hinzufügen in die Zusammensetzung mindestens eines verzweigten primären Alkohols der Formel (I) oder eines Gemisches nach Anspruch 7.

10. Verfahren zum Brechen von Schaum einer Zusammensetzung durch Kontaktieren des Schaums mit mindestens einem verzweigten primären Alkohol der Formel (I) oder einem Gemisch nach Anspruch 7.

11. Verfahren nach einem der Ansprüche 8 bis 10, wobei die Gesamtmenge des (der) verzweigten primären Alkohols (-e) der Formel (I) mindestens 0,01 Gew.-% basierend auf dem Gewicht der Zusammensetzung beträgt.

## Revendications

1. Utilisation d'un alcool primaire ramifié de formule (I), 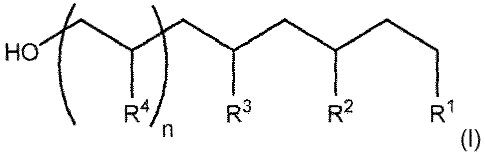 dans laquelle :
- n = 0 ou 1 ;
- R¹, R², R³ et R⁴ sont des groupes alkyle linéaires ou ramifiés, identiques ou différents, comprenant de 1 à 6 atomes de carbone ;
comme antimousse et/ou démousseur.

2. Utilisation selon la revendication 1, dans laquelle lorsque n=0, l'alcool primaire ramifié est un trimère d'alcool primaire, et lorsque n=1, l'alcool primaire ramifié est un tétramère d'alcool primaire, les deux pouvant être obtenus par la condensation de trois et quatre monomères d'alcool primaire respectivement selon une réaction de Guerbet.

3. Utilisation selon la revendication 1 ou 2, dans laquelle des alcools primaires ramifiés de formule (I) avec n=0 et avec n=1, sont utilisés.

4. Utilisation selon l'une quelconque des revendications 1 à 3, dans laquelle R¹, R², R³ et R⁴ sont identiques.

5. Utilisation selon l'une quelconque des revendications 1 à 3, dans laquelle R¹, R², R³ et R⁴ sont indépendamment choisis parmi deux groupes alkyle.

6. Utilisation selon l'une quelconque des revendications 1 à 5, dans laquelle R¹, R², R³ et R⁴, individuellement, comprennent entre 2 et 4 atomes de carbone.

7. Mélange comprenant ou constitué de :
- au moins 25 % en poids de :
- un ou plusieurs alcools primaires ramifiés de formule (I) dans lequel n=0 ; et
- un ou plusieurs alcools primaires ramifiés de formule (I) dans lequel n=1 ;
- moins de 75 % en poids d'un ou plusieurs alcools primaires bêta-alkylés comprenant de 6 à 16 atomes de carbone.

8. Procédé de déstabilisation d'une mousse d'une composition par mise en contact de la mousse avec au moins un alcool primaire ramifié de formule (I) ou avec un mélange selon la revendication 7.

9. Procédé de réduction de formation de mousse d'une composition, par l'ajout dans la composition d'au moins un alcool primaire ramifié de formule (I) ou d'un mélange selon la revendication 7.

10. Procédé de rupture d'une mousse d'une composition, par mise en contact de la mousse avec au moins un alcool primaire ramifié de formule (I) ou avec un mélange selon la revendication 7.

11. Procédé selon l'une quelconque des revendications 8 à 10, dans lequel la quantité totale d'un ou plusieurs alcools primaires ramifiés de formule (I) est d'au moins 0,01 % en poids sur la base du poids de la composition.
